(19) Europäisches Patentamt
European Patent Office
Office européen des brevets

(11) Veröffentlichungsnummer: **0 172 789**
**A2**

(12) **EUROPÄISCHE PATENTANMELDUNG**

(21) Anmeldenummer: **85810372.4**

(22) Anmeldetag: **15.08.85**

(51) Int. Cl.[4]: **C 07 D 231/46**
C 07 C 147/12, C 07 C 147/02
C 07 C 139/00, C 07 D 487/22
C 07 D 231/26, C 09 B 62/503
//(C07D487/22, 259:00, 209:00, 209:00, 209:00)

(30) Priorität: **21.08.84 CH 3993/84**

(43) Veröffentlichungstag der Anmeldung: **26.02.86 Patentblatt 86/9**

(84) Benannte Vertragsstaaten: **BE CH DE FR GB IT LI**

(71) Anmelder: **CIBA-GEIGY AG**
**Klybeckstrasse 141**
**CH-4002 Basel(CH)**

(72) Erfinder: **Schwander, Hansrudolf, Dr.**
**Unterm Schellenberg 189**
**CH-4125 Riehen(CH)**

(54) **Verfahren zur Sulfatierung von Verbindungen, enthaltend Hydroxyäthyl-Gruppen.**

(57) Die Sulfatierung von Verbindungen, welche β-Hydroxyäthylgruppen enthalten, gelingt auf einfache Weise durch Behandeln mit $SO_3$ oder Chlorsulfonsäure in N-Alkylformanilid.

EP 0 172 789 A2

Croydon Printing Company Ltd

CIBA-GEIGY AG
Basel (Schweiz) Case 1-15044/=

# Verfahren zur Sulfatierung von Verbindungen, enthaltend Hydroxyäthyl-Gruppen

Die vorliegende Erfindung betrifft ein Verfahren zur Sulfatierung von Verbindungen, enthaltend Hydroxyäthyl-Gruppen, insbesondere Hydroxyäthylsulfonyl-Gruppen, durch Behandeln mit $SO_3$ oder Chlorsulfonsäure in ausgewählten organischen Lösungsmitteln.

Die Sulfatierung solcher Verbindungen mit $SO_3$ oder Chlorsulfonsäure ist an sich bekannt. Sie erfolgt z.B. in N-Methylpyrrolidon als Lösungsmittel bei einer Temperatur zwischen etwa 20 und 80°C. Dieses Verfahren weist jedoch den Nachteil auf, dass nach beendeter Reaktion und Zerstörung des überschüssigen Sulfatierunsmittels mittels Eiswasser das N-Methylpyrrolidon durch relativ aufwendige Massnahmen, z.B. durch Extraktion mit einem geeigneten Lösungsmittel, aus der wässrigen Lösung abgetrennt werden muss.

Es wurde nun gefunden, das man überraschenderweise die Sulfatierung der genannten Verbindungen in hoher Ausbeute und mit guter Reinheit in spezifischen organischen Lösungsmitteln aus der Klasse der Formanilide durchführen kann. Die Abtrennung der Formanilide von der wässrigen Lösung nach beendeter Reaktion ist infolge der Ausbildung von zwei Phasen sehr einfach durchführbar.

Gegenstand der vorliegenden Erfindung ist somit ein Verfahren zur Herstellung von Verbindungen der Formel I

$$R - CH_2 - CH_2 - O - SO_3H \qquad (I),$$

worin R den Rest einer organischen Verbindung bedeutet, durch Umsetzung einer Verbindung der Formel II

$$R - CH_2 - CH_2 - OH \qquad (II)$$

mit $SO_3$ oder Chlorsulfonsäure in einem organischen Lösungsmittel, dadurch gekennzeichnet, dass als organisches Lösungsmittel eine Verbindung der Formel III

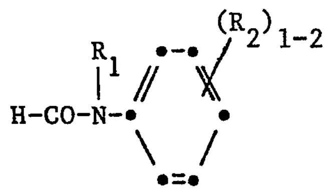

(III)

verwendet wird, worin $R_1$ $C_1$-$C_4$-Alkyl und $R_2$ Wasserstoff, Methyl oder Aethyl bedeutet.

Bevorzugt findet das erfindungsgemässe Verfahren Anwendung zur Sulfatierung von Verbindungen der Formel IV

$$D - SO_2 - CH_2 - CH_2 - OH \qquad (IV)$$

oder der Formel V

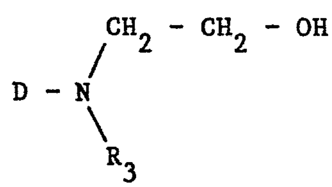

(V)

worin D den Rest eines sulfogruppenhaltigen organischen Farbstoffes oder den Rest einer Diazo- oder Kupplungskomponente und $R_3$ Wasserstoff, $C_1$-$C_4$-Alkyl oder $-CH_2-CH_2OH$ bedeutet.

Bedeutet D den Rest eines sulfogruppenhaltigen organischen Farbstoffes, so handelt es sich z.B. um den Rest eines Farbstoffes der

Mono- oder Polyazo-, Metallkomplexazo-, Anthrachinon-, Phthalocyanin-, Formazan-, Azomethin-, Dioxazin-, Phenazin-, Stilben-, Triphenylmethan-, Xanthen-, Thioxanthon-, Nitroaryl-, Naphthochinon-, Pyrenchinon- oder Perylentetracarbamid-Reihe. Solche Farbstoffe sind bekannt als Reste von Reaktivfarbstoffen mit z.B-Vinylsulfonyl- oder ß-Sulfatoäthylsulfonyl-Gruppen.

Handelt es sich bei D um den Rest einer Diazokomponente, so kommen als Verbindungen der Formel IV z.B. die folgenden in Frage: 1-Amino-4-ß-hydroxyäthylsulfonylbenzol, 1-Amino-3-ß-hydroxyäthylsulfonylbenzol, 1-Amino-2,4-di-(ß-hydroxyäthylsulfonyl)-benzol, 1-Amino-2,4-di-(ß-hydroxyäthylsulfonyl)-5-chlorbenzol, 1-Amino-2-methoxy-5-ß-hydroxyäthylsulfonylbenzol, 1-Amino-4-methoxy-3-ß-hydroxyäthylsulfonylbenzol, 1-Amino-2,5-dimethoxy-4-ß-hydroxyäthylsulfonylbenzol, 1-Amino-2-methoxy-4-ß-hydroxyäthylsulfonyl-5-methylbenzol, 1-Amino-3-ß-hydroxyäthylsulfonyl-6-carboxybenzol, 1-Amino-4-ß-hydroxyäthylsulfonylbenzol-2-sulfonsäure, 1-Amino-5-ß-hydroxyäthylsulfonylbenzol-2,4-disulfonsäure, 1-Amino-2-hydroxy-5-ß-hydroxyäthylsulfonylbenzol, 1-Amino-2-hydroxy-4-ß-hydroxyäthylsulfonylbenzol, 1-Amino-2-hydroxy-5-ß-hydroxyäthylsulfonylbenzol-3-sulfonsäure, 1-Amino-2-brom-4-ß-hydroxyäthylsulfonylbenzol, 1-Amino-2,6-dichlor-4-ß-hydroxyäthylsulfonylbenzol, 1-Amino-2,4-di-(ß-hydroxyäthylsulfonyl)-benzol, 1-Amino-2,4-di-(ß-hydroxyäthylsulfonyl)-5-chlorbenzol, 2-Amino-8-ß-hydroxyäthylsulfonylnaphthalin, 2-Amino-6-ß-hydroxyäthylsulfonylnaphthalin, 2-Amino-6-ß-hydroxyäthylsulfonylnaphthalin-1-sulfonsäure, 2-Amino-8-ß-hydroxyäthylsulfonylnaphthalin-6-sulfonsäure, 2-Amino-6,8-di-(ß-hydroxyäthylsulfonyl)-naphthalin.

Geeignete Verbindungen der Formeln IV oder V, bei denen D den Rest einer Kupplungskomponente bedeutet, sind beispielsweise 1-(4'-ß-Hydroxyäthylsulfonyl)-phenyl-3-methyl-pyrazolon-5, 1-Amino-3-(ß-hydroxyäthylsulfonyl)-benzol, 1-Amino-3-(N,N-di-ß-hydroxyäthylamino)-benzol und 1-Amino-3-(N,N-di-ß-hydroxyäthylamino)-4-methoxybenzol.

Das erfindungsgemässe Verfahren findet vorzugsweise Verwendung zur Sulfatierung von Verbindungen der Formel IV oder V, worin D den Rest eines der oben erwähnten Farbstoffe darstellt, und insbesondere zur Sulfatierung eines Farbstoffes der Formel IV.

Die erfindungsgemässe Reaktion wird in einem Lösungsmittel der Formel III

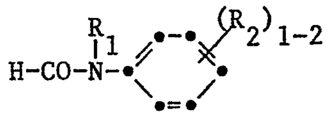

(III)

durchgeführt, worin $R_1$ $C_1$-$C_4$-Alkyl, wie z.B. Methyl, Aethyl, n-Propyl, iso-Propyl oder n-Butyl, und $R_2$ Wasserstoff, Methyl oder Aethyl bedeutet.

Geeignete N-Alkylformanilide sind z.B.
N-Methylformanilid,
N-Aethylformanilid,
N-Propylformanilid,
N-Butylformanilid,
N-Isopropylformanilid,
N-Methyl-2-, -3- oder -4-methylformanilid,
N-Aethyl-2-, -3- oder -4-methylformanilid,
N-Methyl-2-, -3- oder -4-äthylformanilid,
N-Aethyl-2-, -3- oder -4-äthylformanilid,
N-Propyl-3-methylformanilid,
N-Propyl-3-äthylformanilid,
N-Butyl-3-methylformanilid,
N-Butyl-3-äthylformanilid,
N-Methyl-2,5-dimethylformanilid,
N-Aethyl-2,5-dimethylformanilid,
N-Methyl-2,5-diäthylformanilid,
N-Methyl-2,4-dimethylformanilid,
N-Methyl-2,4-diäthylformanilid.

Statt eines einzelnen Formanilids können auch Mischungen verschiedener Formanilide verwendet werden. Die bevorzugten Lösungsmittel der Formel III sind N-Aethylformanilid, N-Aethyl-3-methylformanilid und vor allem N-Methylformanilid.

Als Sulfatierungsmittel verwendet man $SO_3$ oder Chlorsulfonsäure, wobei $SO_3$ als Substanz, als Oleum, z.B. als 20 bis 60 Gew.%iges Oleum, oder als Lösung von $SO_3$ in einem Lösungsmittel der Formel III eingesetzt wird.

Das Sulfatierungsmittel wird in der ein- bis zweifachen, vorzugsweise ein- bis 1,5-fachen molaren Menge, bezogen auf die Hydroxyäthyl-Verbindung, verwendet. Die Reaktionstemperatur liegt zweckmässigerweise zwischen 10 und 120°C, vorzugsweise 20 und 70°C.

Nach beendeter Reaktion wird zunächst wie üblich aufgearbeitet, z.B. indem man die Reaktionsmischung auf Eis giesst. Anschliessend kann das Lösungsmittel leicht abgetrennt werden, z.B. in einem Scheidetrichter, und aus der wässrigen Phase die sulfatierte Verbindung isoliert werden, beispielsweise durch Eindampfen zur Trockne.

Geringe Reste von Lösungsmittel in der wässrigen Phase lassen sich mit einem Extraktionsmittel wie z.B. Toluol oder Methylenchlorid extrahieren. Da die Lösungsmittel der Formel III nur wenig in Wasser löslich sind, benötigt man nur geringe Mengen an Extraktionsmittel im Gegensatz zu dem bekannten Verfahren, in dem als Lösungsmittel N-Methylpyrrolidon verwendet wird.

Die nachstehenden Beispiele dienen zur Erläuterung der Erfindung. Die Temperaturen sind in Grad Celsius angegeben.

Beispiel 1: Zu einem Gemisch, bestehend aus 34 g der Verbindung der Formel

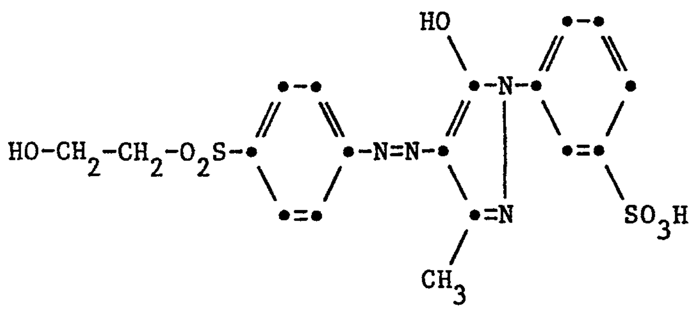

als Natriumsalz und 90 ml N-Methylformanilid, lässt man 8,1 ml Chlorsulfonsäure zutropfen, wobei man die Temperatur unter 40° hält. Man rührt hierauf die Mischung während 3 Stunden bei 40-42°, worauf man sie auf 300 ml Eis giesst. Durch Zugabe von Natriumbicarbonat stellt man den pH-Wert des Gemisches auf 5,5, worauf man dasselbe in einen Scheidetrichter gibt und die N-Methylformanilid-Schicht abtrennt. Man extrahiert die wässrige Phase noch dreimal mit je 15 ml Methylenchlorid, die Methylenchlorid-Auszüge werden mit dem abgetrennten N-Methyl-formanilid vereinigt zwecks Regenerierung durch Destillation. Die wässrige Phase wird in einem Rotationsverdampfer im Wasserstrahlvakuum bei einer Badtemperatur von 40° zur Trockne eingedampft. Es hinterbleiben 52 g eines gelben Pulvers, welches das Natriumsalz des Farbstoffs der Formel

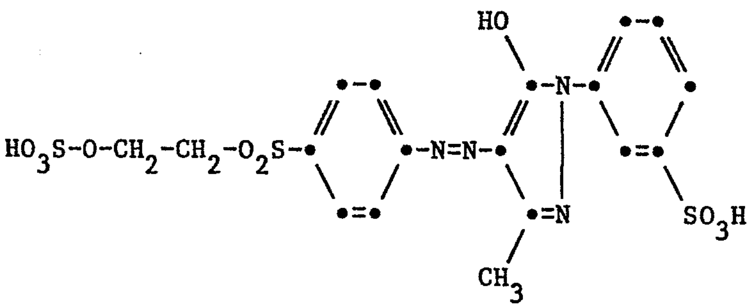

enthält.

Verwendet man anstelle von N-Methylformanilid N-Aethylformanilid, N-Aethyl-3-methylformanilid oder N-Butylformanilid so erhält man ebenfalls den oben beschriebenen Farbstoff mit derselben Ausbeute.

Beispiel 2: Arbeitet man wie im Beispiel 1 beschrieben, verwendet jedoch bei sonst gleichem Vorgehen anstelle des oben aufgeführten Edukts eine äquivalente Menge des Farbstoffs der Formel

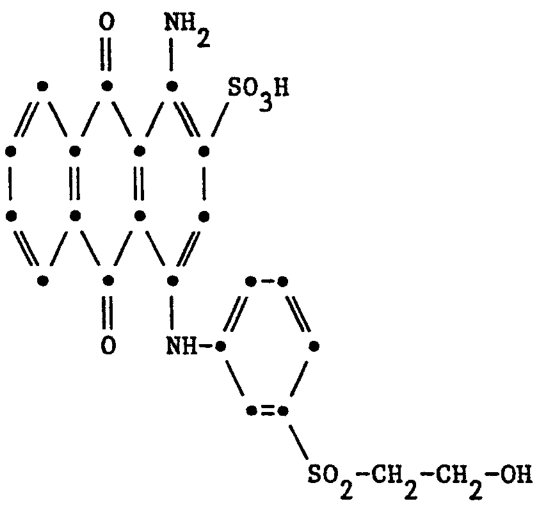

so erhält man in ebenfalls guter Ausbeute den Farbstoff der Formel

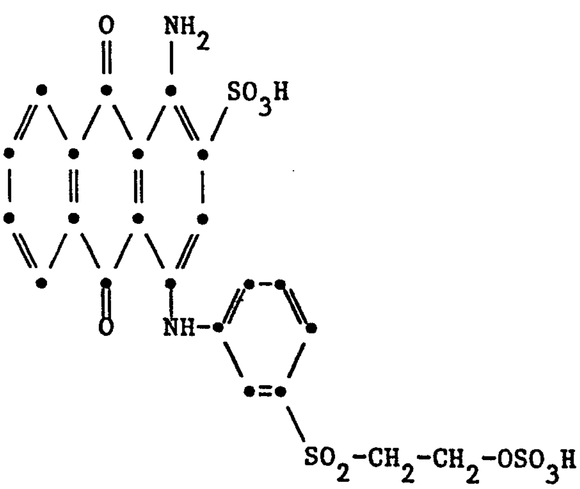

Beispiel 3: Arbeitet man wie im Beispiel 1 beschrieben, verwendet jedoch als Edukt bei gleichem Vorgehen eine äquivalente Menge der Phthalocyaninverbindung der durchschnittlichen Zusammensetzung

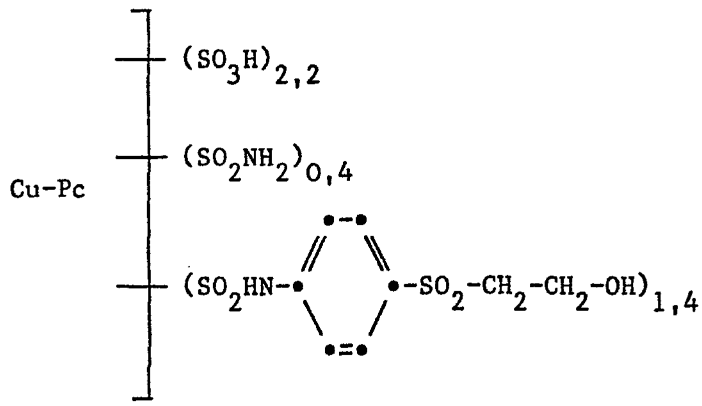

so erhält man den Farbstoff der durchschnittlichen Zusammensetzung entsprechend der Formel

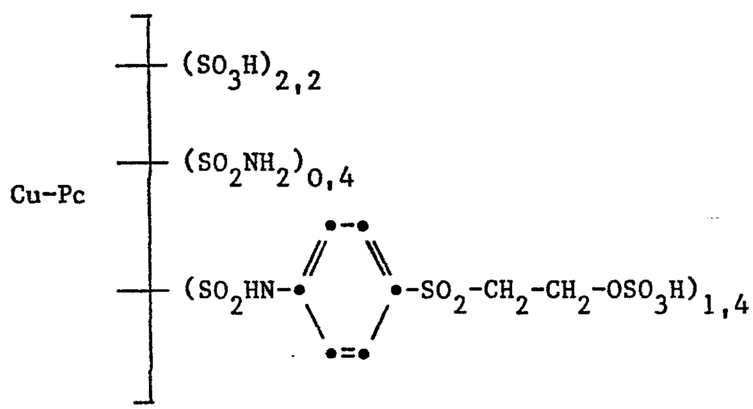

<u>Beispiel 4</u>: Zu einem Gemisch, bestehend aus 9,5 g der Verbindung der Formel

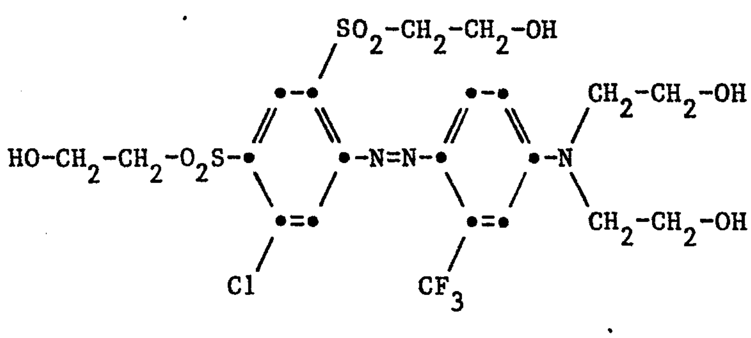

und 40 ml N-Methylformanilid, lässt man bei einer Temperatur von 20-40° 14,6 g Chlorsulfonsäure zutropfen, worauf man die Mischung noch während 4 Stunden bei 43-45° rührt. Man giesst hierauf das Gemisch auf 140 ml einer Eis-Wasser-Mischung und stellt durch Zugabe

von Natriumbicarbonat einen pH-Wert von 5,5 ein. Man trennt das N-Methylformanilid im Scheidetrichter ab und extrahiert die wässrige Phase noch zweimal mit je 15 ml Toluol. Die wässrige Phase wird in einem Rotationsverdampfer im Wasserstrahlvakuum bei einer Badtemperatur von 40° zur Trockne eingedampft, wobei der Farbstoff der Formel

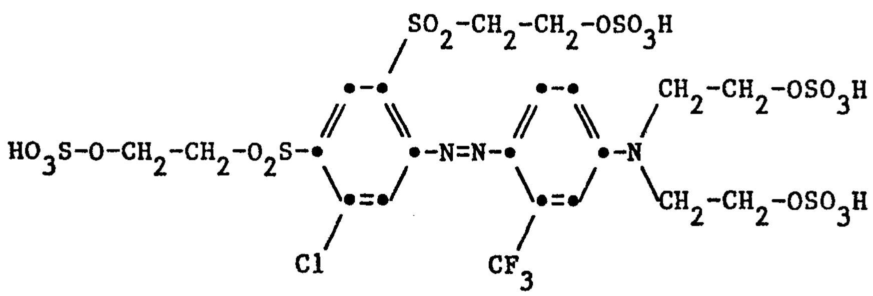

als Natriumsalz in Form eines roten Pulvers erhalten wird. Es hat auch Sulfatierung der Dioxäthylamino-Gruppe stattgefunden.

In analoger Weise kann auch der Farbstoff der Formel

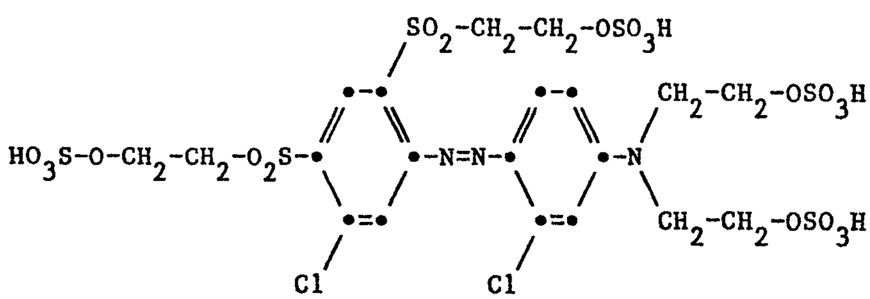

erhalten werden.

Beispiel 5: Zu einem Gemisch, bestehend aus 28,2 g der Verbindung der Formel

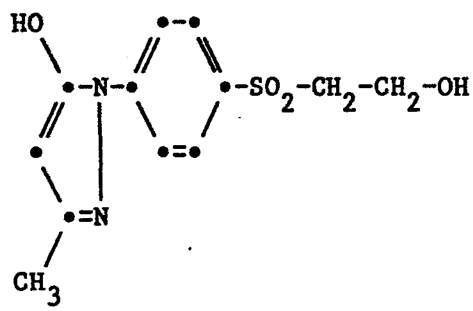

und 60 ml N-Methylformanilid, lässt man bei einer Temperatur von 20-30° innerhalb von etwa 15 Minuten 9,6 ml Chlorsulfonsäure zutropfen, worauf man die Mischung noch während einer Stunde bei 30-32° rührt. Die Aufarbeitung erfolgt analog wie im Beispiel 1 beschrieben. Man erhält die Verbindung der Formel

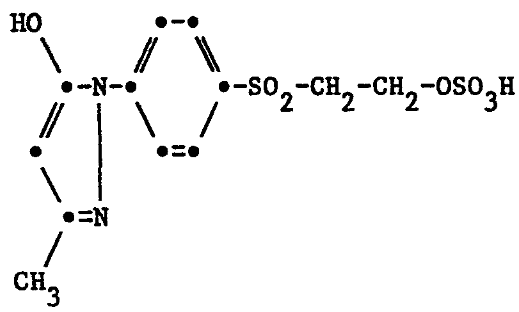

als Natriumsalz in Form eines fast farblosen Pulvers.

Dasselbe Ergebnis erhält man bei Verwendung von 16 g 65%igem Oleum anstelle der Chlorsulfonsäure.

Beispiele 6-16: Verwendet man bei sonst gleichem Vorgehen wie im Beispiel 1 anstelle des oben aufgeführten Edukts äquivalente Mengen der in der folgenden Tabelle aufgeführten Verbindungen, so erhält man ebenfalls die entsprechenden Sulfonsäureester.

Tabelle

| Bsp. | |
|---|---|
| 6 | 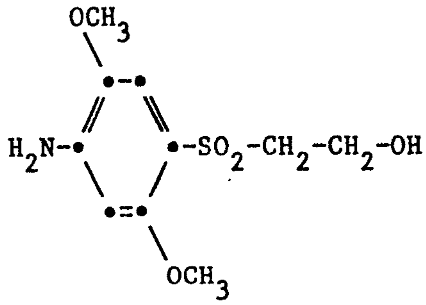 |
| 7 | 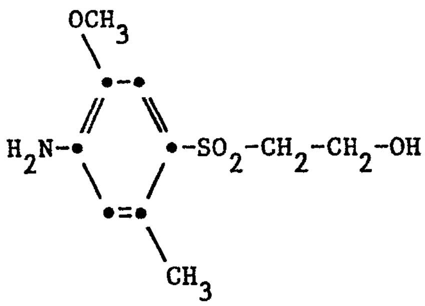 |
| 8 | 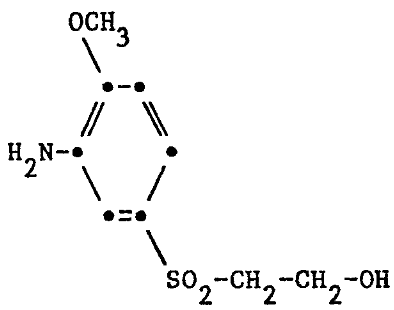 |
| 9 | 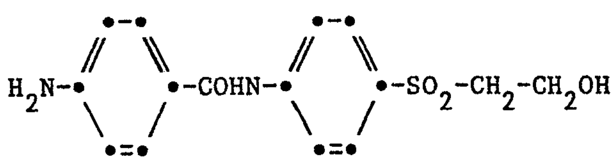 |
| 10 | 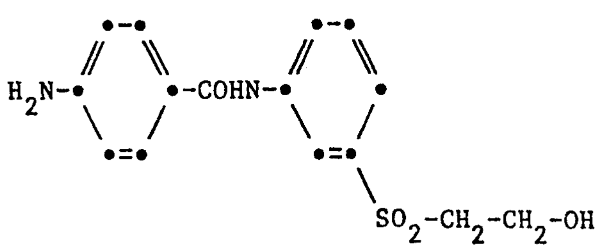 |

| | |
|---|---|
| 11 | 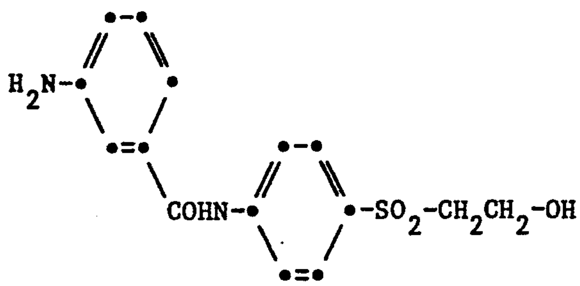 |
| 12 | 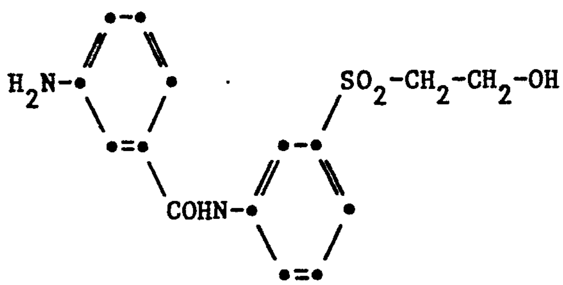 |
| 13 | 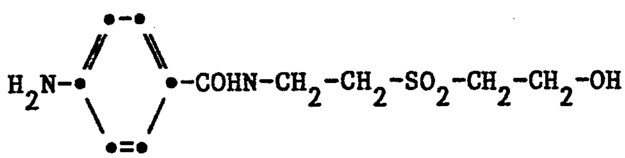 |
| 14 | 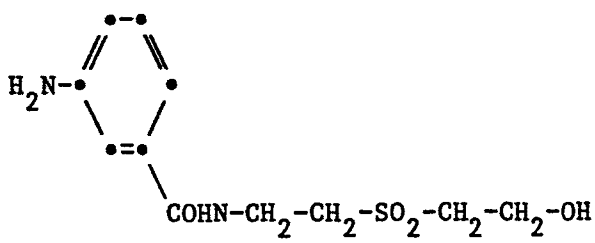 |
| 15 | 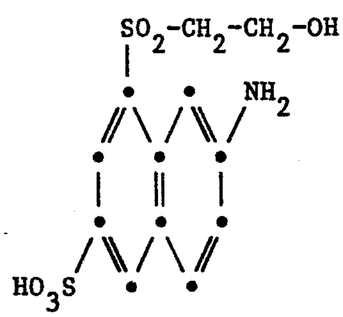 |
| 16 | 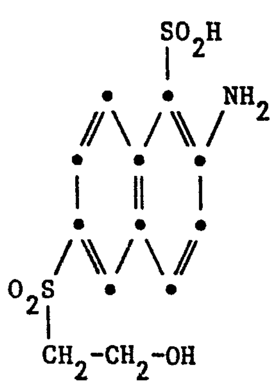 |

Gleiche Ergebnisse wie nach den Beispielen 1 bis 16 erhält man, wenn man die Umsetzung bei 50°C oder 70°C, oder gegebenenfalls bei noch höherer Temperatur, z.B. 100°C oder 120°C, ausführt.

Patentansprüche

1. Verfahren zur Herstellung von Verbindungen der Formel I

$$R - CH_2 - CH_2 - O - SO_3H \qquad (I),$$

worin R den Rest einer organischen Verbindung bedeutet, durch Umsetzung einer Verbindung der Formel II

$$R - CH_2 - CH_2 - OH \qquad (II)$$

mit $SO_3$ oder Chlorsulfonsäure in einem organischen Lösungsmittel, dadurch gekennzeichnet, dass als organisches Lösungsmittel eine Verbindung der Formel III

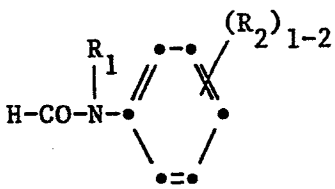

(III)

verwendet wird, worin $R_1$ $C_1$-$C_4$-Alkyl und $R_2$ Wasserstoff, Methyl oder Aethyl bedeutet.

2. Verfahren gemäss Anspruch 1, dadurch gekennzeichnet, dass man eine Verbindung der Formel IV

$$D - SO_2 - CH_2 - CH_2 - OH \qquad (IV)$$

oder der Formel V

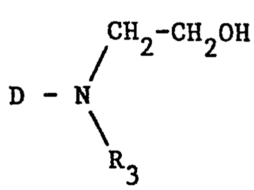

worin D den Rest eines sulfogruppenhaltigen organischen Farbstoffes oder den Rest einer Diazo- oder Kupplungskomponente und $R_3$ Wasserstoff, $C_1$-$C_4$-Alkyl oder $-CH_2-CH_2OH$ bedeutet, mit $SO_3$ oder Chlorsulfonsäure in einer Verbindung der Formel III behandelt.

3. Verfahren gemäss Anspruch 2, dadurch gekennzeichnet, dass man eine Verbindung der Formel IV oder V verwendet, worin D der Rest eines sulfogruppenhaltigen Farbstoffes der Mono- oder Polyazo-, Metallkomplexazo-, Anthrachinon-, Phthalocyanin-, Formazan-, Azomethin-, Dioxazin-, Phenazin-, Stilben-, Triphenylmethan-, Xanthen-, Thioxanthon-, Nitroaryl-, Naphthochinon-, Pyrenchinon- oder Perylentetracarbamid-Reihe ist.

4. Verfahren gemäss Anspruch 3, dadurch gekennzeichnet, dass man eine Verbindung der Formel IV verwendet, worin D die im Anspruch 3 angegebene Bedeutung hat.

5. Verfahren gemäss einem der Ansprüche 1-4, dadurch gekennzeichnet, dass man die Reaktion bei einer Temperatur zwischen 10 und 120°C, vorzugsweise zwischen 20 und 70°C, durchführt.

6. Verfahren gemäss Anspruch 5, dadurch gekennzeichnet, dass man die Reaktion bei einer Temperatur zwischen 30 und 50°C durchführt.

7. Verfahren nach einem der Ansprüche 1-6, dadurch gekennzeichnet, dass das Sulfatierungsmittel in der 1- bis 1,5-fachen molaren Menge, bezogen auf die Verbindung der Formel II, eingesetzt wird.

8. Verfahren gemäss einem der Ansprüche 1-7, dadurch gekennzeichnet, dass als organisches Lösungsmittel der Formel III N-Aethylformanilid, N-Aethyl-3-formanilid oder vor allem N-Methylformanilid verwendet wird.